# EUROPEAN PATENT SPECIFICATION

(11) **EP 1 672 055 B1**
(45) Date of publication and mention of the grant of the patent: **01.10.2008**
(21) Application number: 04773697.0
(22) Date of filing: 01.10.2004
(51) Int. Cl.: C11D 1/10, C07C 231/02, C07C 233/47, A61K 8/18, A61K 8/99

(54) **DETERGENT COMPOSITIONS AND PROCESSES FOR THE PRODUCTION THEREOF**
WASCHMITTEL UND DEREN HERSTELLUNG
COMPOSITIONS DE DETERGENTS ET LEURS PROCEDES DE PRODUCTION

(30) Priority: 03.10.2003 JP 2003345249
(43) Date of publication of application: 21.06.2006
(73) Proprietor: Ajinomoto Co., Inc., Tokyo 104-8315 (JP)
(72) Inventor: HATTORI, Tatsuya,c/o Ajinomoto Co., Inc., Kawasaki-shi, Kanagawa 2108681 (JP)
(74) Representative: Nicholls, Kathryn Margaret
(86) International application number: PCT/JP2004/014900
(87) International publication number: WO 2005/033255

(56) References cited:
- EP-A2- 0 826 766
- WO-A-94/27561
- JP-A- 5 070 418
- JP-A- 5 078 693
- JP-A- 7 062 399
- JP-A- 9 087 666
- JP-A- 11 506 743
- JP-A- 48 010 107
- US-A- 2 880 219
- US-A- 3 836 551

## Description

### Technical Field

The present invention relates to a cleaning composition comprising, as cleaning components, (A) N-long-chain acyl glycine or a salt thereof, (B) N-long-chain acyl glycylglycine or a salt thereof, and (C) N-long-chain acyl glycylglycylglycine or a salt thereof, and further, in addition thereto, (D) a higher fatty acid or a salt thereof, if desired, at a specific weight ratio, and a method for preparing efficiently the cleaning components.

### Background Art

An N-long-chain acyl neutral amino acid salt is a kind of N-long-chain acyl amino acid salt, and is used as a cleaning component for various cleaning compositions, since it has an excellent surface active effect and bacteriostatic effect, and has a low irritation to the skin.

However, when the N-long-chain acyl neutral amino acid salt is solely used as a cleaning agent, the resulting cleaning agent is likely to bring about such problems that it is insufficient in foamability such as lather amount and foam stabilizing property and is lacking in sensory feeling such as smooth feeling and moisture feeling to be provided after cleaning and drying the body or hair. In order to improve those properties, various attempts have been made.

For example, with respect to an N-long-chain acylglycine, there has been invented a method wherein it is added with a small amount of an N-long-chain acylglycylglycine in order to improve its foam stabilizing property and sensory feeling (Japanese Patent No. 2876173). This method, however, has not yet achieved a satisfactory improvement to eliminate a tight feeling of the skin after cleaning, particularly after body cleaning, and is yet insufficient to give sustainable moisture feeling, though it has provided a little improvement.

Also, with respect to an N-long-chain acylalanine, similarly, it needs improving in foamability and smooth feeling for sensory feelings, though it provides moisture feeling, particularly after body cleaning. On the other hand, with respect to a surfactant such as an N-long-chain acylamino acid, there has been proposed a method wherein the surfactant is added with a small amount of an N-long-chain acylalanylalanine salt, in order to provide a cleaning composition which is enhanced in foamability and foam stabilizing property, gives no creaky feeling upon cleaning, has an excellent resistance to hard water, and is excellent in washing-up feeling or wet feeling (Japanese Patent No. 3296062) . The method, however, is insufficient in improvement of smooth feeling after body cleaning.

Therefore, there has been intensively desired a cleaning composition which comprises an N-long-chain acyl neutral amino acid or a salt thereof and will accomplish both foamability such as lather amount and foam stabilizing property and smooth feeling and moisture feeling after drying.

On the other hand, in order to prepare an N-long-chain acyl neutral amino acid or the salt thereof, there has been known a method wherein an amino acid and a fatty acid halide are condensed or reacted with each other in an aqueous solution under an alkaline condition according to the Schotten-Bauman reaction, followed by taking up the reaction product as an acyl amino acid using an acid (For example, JP-A-05-70418 and JP-A-07-157795). This method, which is useful because the reaction is gentle in conditions and proceeds for a relatively short period of time, has nevertheless problems that it requires expensive fatty acid halide as the starting material and by-produces a large amount of inorganic salt which is not easy to remove by purification.

As an example of avoiding such salt by-production, there may be mentioned U.S. Patent 2880219 which discloses a method wherein a fatty acid is reacted with N-methyl taurine or taurine (aminosulfonic acids) to directly amidate. The reaction of the fatty acid with the aminosulfonic acid, however, requires a sever reaction condition of a high temperature of more than 200 °C for a long time.

U.S. Patent 3150156 also discloses a preparation method of an acylmetyltaurine salt. In order to shorten the reaction time and to improve the reaction efficiency, various catalysts have been studied, and however, a lowered temperature of below 200°C has been required to achieve a high yield.

On the other hand, JP-A-2002-234868 discloses a preparation method of an acyltaurine salt. In the method, a metal compound is used as a catalyst to react a fatty acid with a taurine salt, allowing the reaction time to shortern, but the method can not be exempted from a reaction temperature of beyond 190°C, and further, leaves a slight amount of the catalyst in the reaction product. Thus, the reaction mixture per se can not be used directly in a final product.

On the other hand, Published Japanese translation of WO96/39375 (Tokuhyo) No. 506743/'99 discloses, as an example of direct amidation of an amino acid with a fatty acid, a preparation method of the alkali metal salt of an N-acylamino acid, particularly sodium N-acylsarcosinate. This method is more specifically a method wherein the alkaline metal salt of an amino acid is directly reacted with a fatty acid at a high temperature while the water produced during the reaction is continuously removed. The method, however, must be carried out in a non-aqueous system from the outset of the reaction, and needs a complicated procedure such that the amino acid salt and free amino acid should be added individually. In addition, the method has a drawback that to increase the reaction rate and to achieve a high conversion rate require an excessive free amino acid to be added in the reaction mixture of the alkali metal amino acid and the fatty acid, resulting in a surplus of the amino acid left after the reaction.

Under the above-described background art, there has been intensively desired a method for preparing an N-long-chain acyl glycine or a salt thereof, which method can proceed or be carried out under a relatively gentle reaction condition to provide a reaction mixture which can be used directly in a final product.

### Disclosure of the Invention

### [Problems to be Solved by the Invention]

The present invention has been made in the light of the aforementioned background art, and an object thereof is to provide a cleaning composition which is used for cleaning to give abundant foams accompanied with smooth feeling and moisture feeling after cleaning and drying. Another object thereof is to provide an efficient and simple method for preparing an N-long-chain acyl glycine or a salt thereof. Herein, the smooth feeling means the smoothness felt when hair or body is touched with hand. And, herein, the moisture feeling means the state of hair which is free from dryness or drying out, or state of skin which feels fresh and soft.

### [Means for Solving the Problems]

The present inventor has made an intensive study to solve the aforementioned problems, and, as a result, found that the following three components, that is, (A) N-long-chain acyl glycine or a salt thereof, (B) N-long-chain acyl glycylglycine or a salt thereof, and (C) N-long-chain acyl glycylglycylglycine or a salt thereof can be mixed at a specific weight ratio to provide a cleaning composition which brings about abundant foams, is excellent in foam stabilizing property, and furnishes a cleaned and dried body or hair with sensory feeling of both smooth feeling and moisture feeling. Further, the inventor has found that (D) a higher fatty acid having 8 to 22 carbon atoms or a salt thereof is added to those three components at a particular weight ratio to mix, thereby to provide the cleaning composition with enhanced creamy property in foam quality. Furthermore, the inventor has found that glycine, a long-chain fatty acid, and at least one of an alkaline substance selected from sodium hydroxide and potassium hydroxide are mixed, kept with heating, in an inert gas atmosphere if desired, and cleared continuously of the water produced during the reaction, allowing dehydration condensation reaction to proceed at a relatively low temperature for a relatively short time, resulting in the efficient production of an N-long-chain acyl glycine salt accompanied with no catalyst employed. On these findings has been completed the present invention.

Namely, the present invention includes the following embodiments:
(1) A cleaning composition comprising, as cleaning components, (A) N-long-chain acyl glycine or a salt thereof, (B) N-long-chain acyl glycylglycine or a salt thereof, and (C) N-long-chain acyl glycylglycylglycine or a salt thereof, at a weight ratio of (A): (B): (C) = 100: (8 to 50): (0.2 to 18) wherein the long chain acyl group is one or a mixture of carbon chains derivable from a linear or branched alkyl or alkenyl fatty acid having 8 to 22 carbon/atoms.
(2) The cleaning composition of (1) above, wherein the weight ratio of the components is (A): (B): (C) = 100: (9 to 45): (0.4 to 10).
(3) The cleaning composition of (1) or (2) above, which further comprises, as cleaning components, (D) a higher fatty acid having 8 to 22 carbon atoms or a salt thereof.
(4) The cleaning composition of any one of (1) to (3) above, wherein the said cleaning composition is used for body cleaning.
(5) A method for preparing an N-long-chain acyl glycine salt, comprising mixing three components of (a) a saturated or unsaturated linear or branched fatty acid having 8 to 22 carbon atoms, (b) glycine, and (c) at least one of an alkaline substance selected from sodium hydroxide and potassium hydroxide, the molar ratio of (a) to (b) being in the range of 1.5 - 4:1 and subjecting the mixture to heat-dehydration condensation reaction by heating at a maintained temperature of 150 to 190°C while the water produced during the reaction is continuously removed.
(6) The method for preparing an N-long-chain acyl neutral amino acid salt of (5) above, wherein the said heat-dehydration condensation reaction is carried out in an inert gas atmosphere.
(7) The method for preparing an N-long-chain acyl neutral amino acid salt of (5) or (6) above, wherein the said alkaline substance is potassium hydroxide.
(8) A cleaning composition comprising, as the cleaning component, an N-long-chain acyl neutral amino acid salt prepared by the method of any one of (5) to (7) above.
(9) A cleaning composition comprising, as the cleaning component, the reaction mixture per se of an N-long-chain acyl neutral amino acid salt prepared by the method of any one of (5) to (7) above.

### [Effects of the Invention]

The present invention can provide a cleaning composition which brings abundant foams, has an excellent foam stabilizing property, and furnishes a cleaned or dried body or hair with sensory feeling of both smooth feeling and moisture feeling. Further, it can provide a cleaning composition which is enhanced creamy property in foam quality. Furthermore, it can prepare the defined N-long-chain acyl glycine salt efficiently with no catalyst employed through dehydration condensation reaction which can proceed at a relatively low temperature for a short time. Therefore, it can provide a reaction mixture which can be used directly in a final product.

### Best Modes for Carrying Out the Invention

In the following, the present invention will be specifically explained.

Firstly, the cleaning composition of the present invention comprising, as cleaning components, three components (A), (B) and (C), or four components, (D) in addition to (A), (B) and (C) will be explained.

The acyl group of the components: (A) N-long-chain acyl glycine or a salt thereof, (B) N-long-chain acyl glycylglycine or a salt thereof, and (C) N-long-chain acyl glycylglycylglycine or a salt thereof, which are used in the cleaning composition of the present invention, is a carbon chain which is derivable from a linear or branched alkyl or alkenyl fatty acid having 8 to 22 carbon atoms, and may be a single chain or mixed chains. Preferred examples are octanoyl group, decanoyl group, lauroyl group, myristoyl group, palmitoyl group, stearoyl group, oleoyl group, coconut oil fatty acid acyl group, palm oil fatty acid acyl group, palm kernel oil fatty acid acyl group. Glycine is used as a starting material of the above-mentioned three components: (A), (B) and (C) of the cleaning composition of the present invention.

The base as the salt-forming base component, which neutralizes the acyl-neutral amino acid, its dipeptide and its tripeptide of the three components: (A), (B), and (C) in the cleaning composition of the present invention to give their respective corresponding salts, may be one usually used and is not particularly limited. Usable examples are inorganic bases typically represented by alkaline metals such as sodium, potassium, and alkaline earth metals such as magnesium, calcium; ammonia and organic bases typically represented by organic amines such as alkanol amine and basic amino acids such as lysine, and arginine. These can be used solely or in the form of a mixture of two or more thereof.

The method for preparing the components (A), (B), and (C) in the cleaning composition of the present invention is not particularly limited. For example, the component (A) can be prepared according to a usual method such as the Schotten-Bauman reaction of an acyl glycine with a long-chain fatty acid chloride derivable from a long-chain fatty acid. The component (B) can be prepared according to a reaction such as the Schotten-Bauman reaction of an acid chloride derivable from (A) with glycine. Similarly, the component (C) can also be prepared according to a reaction such as the Schotten-Bauman reaction of an acid chloride derivable from (B) with glycine.

The total content of the three components (A), (B) and (C) to be employed in the cleaning composition of the present invention is within a range of from 5 to 100% by weight. From the viewpoint of achieving a sufficient effect, preferable is from 7 to 95% by weight, more preferable is from 10 to 90% by weight. The total content of less than 5% by weight can not provide sufficient sensory feelings.

A weight ratio of the component (A): the component (B): the component (C) in the cleaning composition of the present invention is within a range of 100: (8 to 50): (0.2 to 18). From the viewpoint of achieving a more distinct effect, preferable is 100: (9 to 45): (0.4 to 10), more preferable is 100: (10 to 40): (0.7 to 10), furthermore preferable is 100: (12 to 35): (1 to 10), and particularly preferable is 100: (14 to 25): (1.5 to 5). The component (B) and the component (C), which are in an amount of less than 8 and less than 0.2 respectively, or in an amount of more than 50 and more than 18, fail to give sufficient effects in foamability upon cleaning the body or hair, and in smooth feeling and moisture feeling after drying.

The acylamino acid (A), which is derivable from glycine having no optical active center, has inherently a property to give smooth feeling after drying, and can be added with the aforementioned given amount of (B), particularly the component (C) to bring about additionally the moisture feeling after drying. Glycine is mentioned from the viewpoint of easy availability and processing and high stability.

A higher fatty acid to be blended in the cleaning composition of the present invention can be the same as the linear or branched alkyl or alkenyl fatty acid having 8 to 22 carbon atoms from which the aforementioned long-chain acyl group is derivable.

The component (D), a higher fatty acid or a salt thereof is usually blended to give a ratio relative to a total weight of the components (A), (B) and (C), of (A+B+C): (D) = 100: (30 to 400), and preferably 100: (40 to 300) from the viewpoint of increasing creamy property in foam quality. The ratio of less than 30 or more than 400 brings about insufficient creamy property in foam quality.

The cleaning composition thus prepared, of the present invention is usable for hair and body, and, can be used, for example, as shampoos, rinsing shampoos, conditioning shampoos, facial cleanser, makeup removers, cleansing foams, cleansing powders, cleansing lotions, cleansing creams, hand soaps, bar soaps, mouth washes, shaving foams, and body shampoos. Among them, it is preferably used for body.

Also, of course, there can be blended component(s) usually used in cleaning compositions such as oil solutions, surfactants, gums, antiseptic agents, fragrance agents, UV absorbents, moisture holding agents, physiologically active components, antioxidants, anti-inflammatory agents, antibacterial agents, antiperspirants, chelating agents, neutralizing agents, and pH regulating agents in those cleaning composition, as far as they do not inhibit the effects of the present invention, according to exemplified uses and dosage forms.

Next, a preparing method of the present invention and a cleaning composition using the reaction product obtained according to the preparing method are explained.

The preparing method of the present invention is a preparing method according to claim 5 wherein (a) a long-chain fatty acid, (b) glycine, and (c) one or both of sodium hydroxide and potassium hydroxide are mixed and heated while the water produced during the reaction is removed continuously, thereby to obtain an N-long-chain acylglycine salt. Herein, the continuous removal of the water produced means that the water generated by neutralization with an alkaline substance and the water generated by condensation reaction shall be both positively removed. Specifically, the removal can be achieved by removing together with an inert gas stream, or by removing under reduced pressure.

The long-chain fatty acid to be used as one of the starting materials in the preparing method of the present invention is a saturated or unsaturated linear or branched fatty acid having 8 to 22 carbon atoms. In this connection, this fatty acid can be a fatty acid of one kind, or a so-called mixed fatty acid of two or more kinds of fatty acids. Such a long-chain fatty acid can be the same as the above-mentioned linear or branched alkyl or alkenyl fatty acid having 8 to 22 carbon atoms which has already been explained in connection with the long-chain acyl to be derivable therefrom. Examples are, for instance, saturated linear fatty acids such as octanoic acid, decanoic acid, undecanoic acid, lauric acid, myristic acid, pentadecanoic acid, palmitic acid, stearic acid, and behenic acid; unsaturated linear fatty acids such as oleic acid and linoleic acid; saturated branched fatty acids such as isostearic acid; mixed fatty acids such as coconut oil fatty acid, and palm oil fatty acid. These can be used solely or in the form of a mixture of two or more thereof. Among them, taking into consideration the stability to oxidation during the reaction, the saturated fatty acids are preferable. From the viewpoint of general purpose, particularly preferable are lauric acid, mylistic acid, pentadecanoic acid, palmitic acid, and saturated mixed fatty acids containing these as the main component.

Another starting material used in the preparing method of the present invention is glycine, from the viewpoint of excellent sensory of the reaction product.

A long-chain fatty acid and a glycine are used at a molar ratio of 1.5 to 4 moles of the long-chain fatty acid per one mole of the neutral amino acid, and can be used preferably at a molar ratio of 1.5 to 3 moles from the viewpoint of cost and excellent sensory feelings of the reaction product. Further, from the viewpoint of improvement in reaction yield, the ratio is more preferably 2 to 3 moles. A surplus of fatty acid exists to liquefy the reaction mixture, but the reaction mixture, which has a small fatty acid content to lack in fluidity, if sufficiently blended before heating, allows the reaction to proceed in a solid state.

As the alkaline substance to be used in the preparing method of the present invention, any one or both of sodium hydroxide and potassium hydroxide can be used. Of the two alkaline substances, sodium hydroxide is preferable because of high reaction efficiency. The alkaline substance may be used as it is or in the form of an aqueous solution.

The alkaline substance is used in a molar ratio of equivalent or more to the neutral amino acid, from the viewpoint of reaction efficiency. No reaction takes place if no alkaline substance is used. On the other hand, a surplus of alkaline substance is used to cause uniform mixing to be difficult. Thus, a preferable molar ratio is 1 to 1.5. Moreover, taking into consideration the step for removing an excess fatty acid directly from the reaction mixture by manipulations such as reduction of pressure, the more preferable molar ratio is 1 to 1.3.

The reaction temperature for the dehydration condensation with heating in the preparing method of the present invention is 150 to 190°C, preferably 160 to 180 °C. A temperature of less than 150°C does not allow the reaction to proceed, or needs a lot of time to complete the reaction. A temperature of more than 190°C is not necessary. The reaction is usually completed within 6 hours. A temperature of 160 to 180°C takes about 2 hours to complete the reaction. The water produced during the reaction is removed continuously, as described above.

As the reaction condition for the preparing method of the present invention, the reaction mixture is preferably put in an atmosphere of an inert gas such as nitrogen, or helium, in order to avoid coloration due to oxidation.

The N-long-chain acyl glycine salt prepared according to the preparing method of the present invention is in the form of the sodium salt and/or the potassium salt in the reaction mixture due to the used alkaline substance. The reaction mixture can be used as it is directly, or after purified, as a cleaning component for various cleaning compositions. The purification method is not particularly limited, and a method conducted generally in this field can be utilized. For example, there is a method wherein the reaction is completed followed by distilling off an excess fatty acid under a reduced pressure, or a method wherein the reaction mixture is neutralized with a mineral acid such as hydrochloric acid or sulfuric acid to obtain the N-long-chain acyl neutral amino acid in the free form, and then an excess fatty acid can be removed. In addition, the resulting free acid is neutralized with a proper base to prepare a desired N-long-chain acyl neutral amino acid salt. The reaction mixture is particularly preferably used directly as a cleaning component for various cleaning compositions, from the viewpoint that the reaction solution can be, as it is, a product accompanied with no waste solution to drain and can provide a cleaning composition which allows abundant foams upon cleaning and gives refreshing feeling and moisture feeling after cleaning, particularly after body cleaning.

The cleaning composition, wherein such N-long-chain acyl neutral amino acid salt is used as a cleaning component, is usable for hair and body in the same as has been explained above, and can be used, for example, as shampoos, rinsing shampoos, conditioning shampoos, facial cleanser, makeup removers, cleansing foams, cleansing powders, cleansing lotions, cleansing creams, hand soaps, bar soaps, mouth washes, shaving foams, and body shampoos. Among them, it is preferably used for body.

Also, in the same as has been explained above, various components usually used in cleaning compositions such as oil solutions, surfactants, gums, antiseptic agents, flagrance agents, UV absorbents, moisture holding agents, physiologically active components, antioxidants, anti-inflammatory agents, antibacterial agents, antiperspirants, chelating agents, neutralizing agents, and pH regulating agents, can be of course blended in those cleaning compositions, as far as they do not inhibit the effects of the present invention, according to exemplified uses and dosage forms.

### [Example]

In the following, the present invention will be more specifically explained with reference to Examples and Comparative Examples, but is not limited by these Examples.

### Examples 1 to 6 and Comparative Examples 1 to 3

### (Foaming and sensory evaluation of the glycine type)

Cleaning components were mixed at each proportion shown in Table 1 below, and then prepared into an aqueous solution containing a total content of 10% by weight of the components therein. Each of the aqueous solutions was diluted by 40 times (the cleaning components having a concentration of 0.25%), agitated with a mixer at a temperature of 30°C, and then measured for lather amounts at 1 minute and 5 minutes after the agitation. Form retaining rate is indicated in the percentage of a lather amount at 5 minutes after the mixer agitation based on the lather amount at 1 minute after the mixer agitation. In Example 1, the reaction mixture obtained in Preparation Example 9 as mentioned hereinafter was cleared of the fatty acid to obtain a mixture of the acylamino acid and the acylpeptide. Both the acylamino acid and the acylpeptide were then changed to their respective sodium salts for use.

Lather amounts in Table 1 were evaluated on the basis of the lather amounts at 1 minute after the mixer agitation (1 minute lather amount), i.e., ○○: 285 ml or more, ○: 270 ml or more and less than 285 ml, Δ: 255 ml or more and less than 270 ml, and ×: less than 255 ml. Foam stability was evaluated on the basis of foam retaining rate, i.e., ○○: 80% or more, ○: 75% or more and less than 80%, Δ: 65% or more and less than 75%, and ×: less than 65%.

Foam quality and Feelings after dried in the same table were the results obtained by hand washing evaluation by five professional panelists. The evaluation was done by calculating an average value on the basis of the following standards (a) and (b), and an average of 4.5 or more was regarded as very good (○○), 3.5 to 4.4 was regarded as good (○), 2.5 to 3.4 was regarded as usual (Δ), 2.4 or less was regarded as bad (×).
(a) Foam quality (creamy)
   5 : Very creamy
   4 : Somewhat creamy
   3 : Usual
   2 : Somewhat rough
   1 : Rough
(b) Smooth feeling after dried / moisture feeling after dried
   5 : Very smooth / very moisture
   4 : Somewhat smooth / somewhat moisture
   3 : Usual
   2 : Somewhat bad smooth feeling / somewhat bad moisture feeling
   1 : No smooth feeling / no moisture feeling

**Table 1**

| | Example 1 | Example 2 | Example 3 | Example 4 | Example 5 | Example 6 | Comp. Example 1 | Comp. Example 2 | Comp. Example 3 |
|---|---|---|---|---|---|---|---|---|---|
| Lauroyl Gly Na | 100 | 100 | 100 | 100 | 100 | 100 | 100 | 100 | 100 |
| Lauroyl GlyGly Na | 15 | 15 | 34 | 15 | 20 | 25 | 0 | 6 | 55 |
| Lauroyl GlyGlyGly Na | 2 | 10 | 1 | 2 | 5 | 7 | 0 | 0 | 20 |
| Lauric acid Na | 0 | 0 | 0 | 50 | 190 | 530 | 0 | 0 | 0 |
| 1 min. lather amount (ml) 5 min. lather amount (ml) | 280 | 270 | 275 | 300 | 285 | 280 | 270 | 270 | 260 |
| | 235 | 225 | 230 | 265 | 245 | 235 | 150 | 200 | 220 |
| Foam retaining rate (%) | 84 | 83 | 84 | 88 | 86 | 84 | 56 | 74 | 85 |
| Lather amount evaluation | ○ | ○ | ○ | ○○ | ○○ | ○ | ○ | ○ | Δ |
| Foam stability evaluation | ○○ | ○○ | ○○ | ○○ | ○○ | ○○ | × | Δ | ○○ |
| Foam quality (creamy) | ○ | ○ | ○ | ○○ | ○○ | ○○ | × | Δ | ○ |
| Smooth feeling after dried | ○○ | ○○ | ○○ | ○○ | ○○ | ○○ | ○ | ○○ | ○○ |
| Moisture feeling after dried | ○○ | ○○ | ○○ | ○○ | ○○ | ○ | × | × | ○○ |

### Preparation Example 1

5.0 g of glycine (66.7 mmole), 40.0 g of lauric acid (200 mmole) and 9.88 g of 27% sodium hydroxide solution (66.7 mmole) were mixed and then heated in a nitrogen gas stream at 180°C for 1.5 hours. During the heating, the reaction mixture was in the state of liquid. The water produced during the reaction was continuously removed as water vapor together with the nitrogen gas stream. After cooling, pale-yellowish solid was obtained. The reaction mixture was quantitatively determined by HPLC, regarding the acylated forms, i.e., the lauroylglycine and laurolyglycylglycine, the non-acylated forms, i.e., the glycylglycine and glycyldiketopiperazine, and the unreacted glycine. As a result, the reaction efficiency (yield of the acylated forms) was 91.8% by mole, and the overall recovering rate of the glycine present in the mixture (including the unreacted glycine) was 97.1%.

### Preparation Examples 2 to 8

Glycine, a fatty acid having a varying carbon chain length (different number of carbon atoms) and an alkaline substance shown in the following Table 3 were used to react in the same manner as in Preparation Example 1, except that the fatty acid and the alkaline substance were reacted in their various amounts (in molar ratio to the glycine) and at various reaction temperatures. The results are also shown in the following Table 3. Note that the contents of the dipeptide and the tripeptide were shown as their relative weight values to the acylamino acid (the acylamino acid being assumed to be 100).

**Table 3**

| | | Preparation Example 1 | Preparation Example 2 | Preparation Example 3 | Preparation Example 4 | Preparation Example 5 | Preparation Example 6 | Preparation Example 7 | Preparation Example 8 |
|---|---|---|---|---|---|---|---|---|---|
| Amino acid | | Glycine | Glycine | Glycine | Glycine | Glycine | Glycine | Glycine | Glycine |
| Fatty acid (Carbon atom number) | | C12 | C12 | C12 | C12 | C12 | C14 | C12 | C12 |
| (Equivalent) | | 3.0 | 3.0 | 3.0 | 3.0 | 2.5 | 2.5 | 2.0 | .3.0 |
| Alkali (Equivalent) | | NaOH | NaOH | KOH | KOH | KOH | KOH | KOH 1.2 | KOH |
| | | 1.0 | 1.2 | 1.2 | 1.2 | 1.0 | 1.2 | | 1.4 |
| Reaction temp. (°C) | | 180 | 170 | 170 | 150 | 170 | 170 | 180 | 170 |
| Reaction time (hr) | | 1.5 | 1.5 | 1.5 | 6.0 | 1.5 | 1.5 | 2.0 | 2.0 |
| State of Reaction mixture | | liquid | liquid | liquid | liquid | liquid | liquid | solid | liquid |
| Color of reaction product | | pale | pale | pale | pale | pale | pale | pale | pale |
| | | yellowish | yellowish | yellowish | yellowish | yellowish | yellowish | yellowish | yellowish |
| Reaction yield (%) | | 91.8 | 90.4 | 94.9 | 94.5 | 94.3 | 94.0 | 90.8 | 95.0 |
| Recovery yield(%) | | 97.1 | 97.0 | 98.2 | 98.0 | 96.4 | 98.6 | 95.0 | 97.5 |
| Content | Dipeptide | 24.0 | 18.0 | 18.0 | 18.0 | 22.7 | 17.2 | 16.9 | 14.7 |
| | Tripeptide | 3.5 | 2.2 | 2.3 | 1.7 | 3.2 | 2.1 | 2.3 | 1.6 |

### Formulation Example 1 <Solid cleaning agent>

A solid cleaning composition was prepared according to the formulation shown in the following Table 4. The solid cleaning agent thus obtained was excellent in foamability and evaluated by hand washing to provide performance to give both moisture feeling and smooth feeling. The numerical values in the Table represent a percent by weight.

**Table 4**

| | |
|---|---|
| Reaction product of Preparation Example 6 | 35.0 |
| Reaction product of Preparation Example 7 | 25.0 |
| Sodium N-stearoyl-L-glutamate | 15.0 |
| Sodium salt of coconut oil fatty acid | 5.0 |
| Cetanol | 5.0 |
| Glycerin | 5.0 |
| Sodium hydroxide | 3.5 |
| EDTA·2Na | 0.2 |
| Titanium oxide | 0.1 |
| Methylparaben | 0.1 |
| Water | remainder |
| Total | 100.0 |

### Industrial Applicability

Cleaning compositions, including shampoos, rinsing shampoos, conditioning shampoos, facial cleanser, makeup removers, cleansing foams, cleansing powders, cleansing lotions, cleansing creams, hand soaps, bar soaps, mouth washes, shaving foams, and body shampoos, which can provide abundant foams, are excellent in foam stabilizing property, and allow sensory feelings of both smooth feeling and moisture feeling after cleaning and drying a body or hair, can be provided.

## Claims

1. A cleaning composition comprising 5 to 100% by weight of a mixture of the following components (A), (B), and (C), the weight ratio of (A) to (B) to (C) in said mixture lying in the range 100: (8 to 50) : (0.2 to 18) :
(A) N-long chain acyl glycine or a salt thereof:
(B) N-long chain acyl glycylglycine or a salt thereof:
(C) N-long chain acyl glycylglycylglycine or a salt thereof;
wherein said long chain acyl group is one, or a mixture, of carbon chains derivable from a linear or branched alkyl or alkenyl fatty acid having 8 to 22 carbon atoms.

2. The cleaning composition according to claim 1, wherein the weight ratio of components is (A) : (B) : (C) = 100: (9 to 45) : (0.4 to 10).

3. The cleaning composition according to claim 1 or 2, which further comprises, as a cleaning component, (D) a higher fatty acid having 8 to 22 carbon atoms or salt thereof.

4. The cleaning composition according to any one of claims 1 to 3, for use in body cleaning.

5. A method for preparing an N-long-chain acyl glycine salt, which comprises mixing (a) a saturated or unsaturated linear or branched fatty acid having 8 to 22 carbon atoms, (b) glycine, and (c) at least one of an alkaline substance selected from sodium hydroxide and potassium hydroxide, the molar ratio of (a) to (b) being in the range of 1.5-4 : 1, and subjecting the mixture to heat-dehydration condensation reaction by heating at a maintained temperature of 150 to 190°C while the water produced during the reaction is continuously removed.

6. The method according to claim 5 wherein the alkaline substance is used in the form of an aqueous solution.

7. The method for preparing an N-long-chain acyl glycine salt according to claim 5 or 6, wherein the said heat-dehydration condensation reaction is carried out in an inert gas atmosphere.

8. The method for preparing an N-long-chain glycine salt according to any of Claims 5 to 7, wherein the said alkaline substance is potassium hydroxide.

9. A cleaning composition comprising, as the cleaning component, an N-long-chain acyl glycine salt prepared by the method according to any one of claims 5 to 8.

10. A cleaning composition comprising, as a cleaning component, the reaction mixture per se of an N-long-chain acyl glycine salt prepared by the method according to any one of claims 5 to 8.

## Patentansprüche

1. Reinigungszusammensetzung, enthaltend 5 bis 100 Gew.-% eines Gemischs der folgenden Komponenten (A), (B) und (C), wobei das Gewichtsverhältnis von (A) zu (B) zu (C) des Gemischs im Bereich von 100:(8 bis 50):(0,2 bis 18) liegt:
(A) N-Langkettenacylglycin oder ein Salz davon;
(B) N-Langkettenacylglycylglycin oder ein Salz davon;
(C) N-Langkettenacylglycylglycylglycin oder ein Salz davon;
worin die Langkettenacylgruppe eine von oder ein Gemisch von Kohlenstoffketten ist, welche von einer linearen oder verzweigten Alkyl- oder Alkenylfettsäure mit 8 bis 22 Kohlenstoffatomen abgeleitet sind.

2. Reinigungszusammensetzung nach Anspruch 1, wobei das Gewichtsverhältnis der Komponenten (A):(B):(C) = 100:(9 bis 45):(0,4 bis 10) ist.

3. Reinigungszusammensetzung nach Anspruch 1 oder 2, welche weiterhin als eine Reinigungskomponente enthält: (D) eine höhere Fettsäure mit 8 bis 22 Kohlenstoffatomen oder ein Salz davon.

4. Reinigungszusammensetzung nach einem der Ansprüche 1 bis 3 zur Verwendung für die Körperreinigung.

5. Verfahren zur Herstellung eines N-Langkettenacylglycinsalzes, umfassend Mischen (a) einer gesättigten oder ungesättigten linearen oder verzweigten Fettsäure mit 8 bis 22 Kohlenstoffatomen, (b) Glycin und (c) mindestens einer alkalischen Substanz, die aus Natriumhydroxid und Kaliumhydroxid ausgewählt ist, wobei das Molverhältnis von (a) zu (b) im Bereich von 1,5-4:1 ist, und Unterziehen des Gemischs einer Hitzeentwässerungskondensationsreaktion durch Erhitzen bei einer aufrecht erhaltenen Temperatur von 150 bis 190 °C, während das bei der Reaktion erzeugte Wasser kontinuierlich entfernt wird.

6. Verfahren nach Anspruch 5, worin die alkalische Substanz in Form einer wässrigen Lösung verwendet wird.

7. Verfahren zur Herstellung eines N-Langkettenacylglycinsalzes nach Anspruch 5 oder 6, worin die Hitzeentwässerungskondensationsreaktion in einer Inertgasatmosphäre durchgeführt wird.

8. Verfahren zur Herstellung eines N-Langkettenglycinsalzes nach einem der Ansprüche 5 bis 7, worin die alkalische Substanz Kaliumhydroxid ist.

9. Reinigungszusammensetzung, welche als Reinigungskomponente ein N-Langkettenacylglycinsalz enthält, welches nach dem Verfahren nach einem der Ansprüche 5 bis 8 hergestellt wurde.

10. Reinigungszusammensetzung, welche als Reinigungskomponente das Reaktionsgemisch per se eines N-Langkettenacylglycinsalzes enthält, welches durch das Verfahren nach einem der Ansprüche 5 bis 8 hergestellt wurde.

## Revendications

1. Composition de nettoyage comprenant 5 à 100 % en poids d'un mélange des composants suivants (A), (B) et (C), le rapport en poids de (A) sur (B) sur (C) dans ledit mélange se trouvant dans la plage de 100 : (8 à 50) : (0,2 à 18) :
(A) N-acyl glycine à longue chaîne ou un sel de celle-ci ;
(B) N-acyl glycylglycine à longue chaîne ou un sel de celle-ci ;
(C) N-acyl glycylglycine à longue chaîne ou un sel de celle-ci ;
dans laquelle ledit groupe acyle à longue chaîne est l'une, ou un mélange, de chaînes carbonées pouvant être dérivées d'un acide gras alkyle ou alcényle linéaire ou ramifié comportant de 8 à 22 atomes de carbone.

2. Composition de nettoyage selon la revendication 1, dans laquelle le rapport en poids des composants est (A) : (B) : (C) = 100 : (9 à 45) : (0,4 à 10).

3. Composition de nettoyage selon la revendication 1 ou 2, qui comprend en outre comme composant de nettoyage, (D) un acide gras supérieur comportant de 8 à 22 atomes de carbone ou un sel de celui-ci.

4. Composition de nettoyage selon l'une quelconque des revendications 1 à 3, pour une utilisation dans l'hygiène corporelle.

5. Procédé de préparation d'un sel de N-acyl glycine à longe chaîne, qui comprend le mélange (a) d'un acide gras linéaire ou ramifié saturé ou insaturé comportant de 8 à 22 atomes de carbone, (b) de la glycine et (c) au moins l'une d'une substance alcaline choisie parmi l'hydroxyde de sodium et l'hydroxyde de potassium, le rapport molaire de (a) sur (b) étant dans la plage de 1,5 à 4 : 1, et en soumettant le mélange à une réaction de condensation par déshydratation thermique en chauffant à une température maintenue de 150 à 190 °C tout en retirant en continu l'eau produite pendant la réaction.

6. Procédé selon la revendication 5, dans lequel la substance alcaline est utilisée sous la forme d'une solution aqueuse.

7. Procédé de préparation d'un sel de N-acyl glycine à longue chaîne selon la revendication 5 ou 6, dans lequel ladite réaction de condensation par déshydratation thermique est réalisée dans une atmosphère de gaz inerte.

8. Procédé de préparation d'un sel de N-glycine à longue chaîne selon l'une quelconque des revendications 5 à 7, dans lequel ladite substance alcaline est l'hydroxyde de potassium.

9. Composition de nettoyage comprenant, comme composant de nettoyage, un sel de N-acyl glycine à longue chaîne préparé par le procédé selon l'une quelconque des revendications 5 à 8.

10. Composition de nettoyage comprenant, comme composant de nettoyage, le mélange de réaction en soi d'un sel de N-acyl glycine à longue chaîne préparé par le procédé selon l'une quelconque des revendications 5 à 8.
